(19) Europäisches Patentamt
European Patent Office
Office européen des brevets

(11) Veröffentlichungsnummer: **0 104 572**
**A1**

# (12) EUROPÄISCHE PATENTANMELDUNG

(21) Anmeldenummer: **83109251.5**

(22) Anmeldetag: **19.09.83**

(51) Int. Cl.³: **C 07 D 233/60**
C 07 D 235/06, C 07 D 231/12
C 07 D 249/08, C 07 D 295/08
C 07 D 317/22, C 07 D 405/12
C 07 C 43/313, A 61 K 31/40
A 61 K 31/41, A 61 K 31/415

(30) Priorität: **25.09.82 DE 3235589**

(43) Veröffentlichungstag der Anmeldung:
**04.04.84 Patentblatt 84/14**

(84) Benannte Vertragsstaaten:
**AT BE CH DE FR GB IT LI NL SE**

(71) Anmelder: **HOECHST AKTIENGESELLSCHAFT**
**Postfach 80 03 20**
**D-6230 Frankfurt am Main 80(DE)**

(72) Erfinder: **Blume, Ernst, Dr.**
**Im Hainpfad 10**
**D-6232 Bad Soden am Taunus(DE)**

(72) Erfinder: **Granzer, Ernold, Dr. Dr.**
**Falkensteiner Strasse 24**
**D-6233 Kelkheim (Taunus)(DE)**

(54) **Benzylether von Phenol-Mannich-Basen, Verfahren zu ihrer Herstellung, ihre Verwendung sowie pharmazeutische Präparate auf Basis dieser Verbindungen, Zwischenprodukte und Verfahren zu deren Herstellung.**

(57) Benzylether von Phenol-Mannich-Basen der Formel I

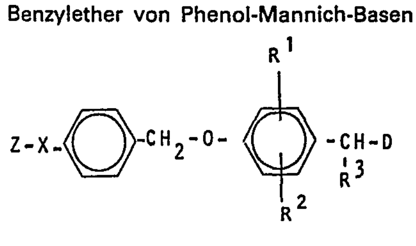

I

worin D, $R^1$, $R^2$, $R^3$, X und Z die angegebenen Bedeutungen haben, deren physiologisch verträgliche Salze, Verfahren zu ihrer Herstellung, pharmazeutische Präparate auf Basis dieser Verbindungen sowie ihre Verwendung als Arzneimittel betreffen die Erfindung. Ferner betrifft die Erfindung Zwischenprodukte der Formel III'

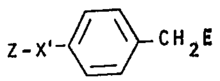

III'

worin, E, Z und X' die angegebenen Bedeutungen haben und Verfahren zu ihrer Herstellung.

EP 0 104 572 A1

Croydon Printing Company Ltd.

# Benzylether von Phenol-Mannich-Basen, Verfahren zu ihrer Herstellung, ihre Verwendung sowie pharmazeutische Präparate auf Basis dieser Verbindungen, Zwischenprodukte und Verfahren zu deren Herstellung

Die vorliegende Erfindung betrifft neue Benzylether von Phenol-Mannich-Basen, Verfahren zu deren Herstellung, diese enthaltende Arzneimittel und ihre Verwendung zur Behandlung von Hyperlipidämien und Arteriosklerose.

In den deutschen Offenlegungsschriften 24 18 502, 25 10 130, 25 10 781, 30 01 762 und der BE-PS 883 665 werden Benzylether mit Wirkung gegen Mikroorganismen und als Thromboxan-Synthetase Inhibitoren beschrieben.

In J. Med. Chem. 18, (1975), S. 833-836 wird über hypolipidämische Effekte von 1-Benzylimidazolen berichtet, deren Wirkung und Verträglichkeit jedoch nicht befriedigend ist.

Der Erfindung liegt die Aufgabe zugrunde, ein zur Behandlung der Hyperlipidämie und Arteriosklerose geeignetes Präparat zur Verfügung zu stellen, das neben einer guten Wirksamkeit auch eine gute Verträglichkeit zeigt.
Überraschenderweise wurde gefunden, daß Benzylether von Phenol-Mannich-Basen der Formel I

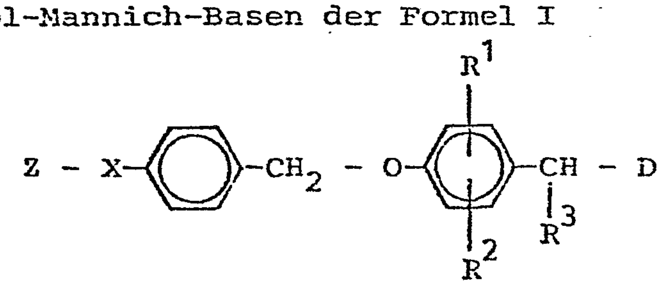

I

in der
$R^1$ und $R^2$ gleich oder verschieden sind und jeweils Wasserstoff, $C_1$-$C_4$-Alkyl, $C_5$-$C_6$-Cycloalkyl, $C_1$-$C_4$-Alkoxy, Allyl oder Halogen bedeuten,

$R^3$ Wasserstoff oder $C_1$-$C_4$-Alkyl bedeutet,
D entweder
a) einen Rest der Formel D (a),

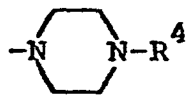 D (a)

in der
$R^4$ Wasserstoff, $C_1$-$C_8$-Alkyl, Allyl, Hydroxyethyl, $C_1$-$C_5$-Alkanoyl, $C_1$-$C_4$-Alkylsulfonyl, $C_1$-$C_4$-Alkoxycarbonyl, Di-($C_1$-$C_4$)-alkylaminocarbonyl oder eine gegebenenfalls 1 oder 2 Substituenten tragende Phenylgruppe bedeutet, wobei die Substituenten gleich oder verschieden sind und jeweils Halogen, $C_1$-$C_4$-Alkyl, $C_1$-$C_4$-Alkoxy oder Trifluormethyl bedeuten, oder
b) einen Pyrrolidin-1-yl-, Piperidin-1-yl-, Morpholin-4-yl-, Pyrazol-1-yl-, Imidazol-1-yl-, 1,2,4-Triazol-1-yl- oder einen Benzimidazol-1-yl-Rest bedeutet,

X 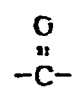 , 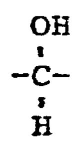 , 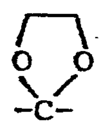 oder 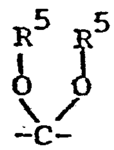 bedeutet,

wobei $R^5$ $C_1$-$C_4$-Alkyl ist,
Z a) einen Rest der Formel Z(a)

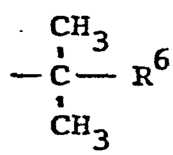 Z (a)

in der $R^6$ H, $C_1$-$C_4$-Alkyl, Halogen oder $-CH_2$-Halogen bedeutet, darstellt oder
b) eine gegebenenfalls 1 oder 2 Substituenten tragende Phenylgruppe bedeutet, wobei die Substituenten gleich oder verschieden sind und jeweils Halogen, Hydroxy, $C_1$-$C_4$-Alkyl, $C_1$-$C_4$-Alkoxy oder Trifluormethyl bedeuten, und ihre physiologisch verträgliche Salze mit Säuren, eine bessere lipidsenkende Wirkung und Verträglichkeit

zeigen als die beschriebenen 1-Benzylimidazole, von denen sie sich insbesondere durch die Art der Substituenten im Benzylether-Teil unterscheiden.

Die Erfindung betrifft daher Verbindungen der Formel I, die in verschiedenen stereoisomeren Strukturen vorliegen sowie deren physiologisch verträgliche Salze.

In den vorstehenden und folgenden Ausführungen ist unter dem Ausdruck
"$C_1$-$C_4$-Alkyl" ein unverzweigter oder verzweigter Kohlenwasserstoffrest mit 1 - 4 Kohlenstoffatomen, wie z.B. der Methyl-, Ethyl-, Propyl-, 1-Methylethyl-, Butyl- 2-Methylpropyl- oder 1,1-Dimethylethyl-Rest,
unter dem Ausdruck
"$C_1$-$C_8$-Alkyl" ein unverzweigter oder verzweigter Kohlenwasserstoffrest mit 1 - 8 Kohlenstoffatomen, wie z.B. die vorstehend genannten Reste oder eine Pentyl-, Hexyl-, Heptyl-, Oktyl- oder 1,1-Dimethyl-3,3-dimethyl-butyl-Gruppe,
unter dem Ausdruck
"$C_1$-$C_4$-Alkoxy" eine Alkoxygruppe, deren Kohlenwasserstoffrest die unter dem Ausdruck "$C_1$-$C_4$-Alkyl" angegebene Bedeutung hat,
unter dem Ausdruck
"$C_1$-$C_5$-Alkanoyl" ein unverzweigter oder verzweigter Alkanoylrest mit 1 bis 5 Kohlenstoffatomen, wie die Formyl-, Acetyl-, Porpanoyl-, 2-Methylpropanoyl-, Butanoyl-, 2-Methylbutanoyl- und 2,2-Dimethylpropanoyl-Gruppe,
unter dem Ausdruck
"Halogen" ein Jod- oder vorzugsweise ein Brom-, Chlor- oder Fluoratom zu verstehen.

Bevorzugt sind Verbindungen der Formel I, in denen D Pyrazol-1-yl, Imidazol-1-yl, 1,2,4-Triazol-1-yl oder einen Rest der Formel D (a) bedeutet, in der $R^4$ Wasserstoff, Methyl, Acetyl, Methoxycarbonyl, Dimethylaminocarbonyl,

Methylsulfonyl oder eine gegebenenfalls mono-substituierte Phenylgruppe bedeutet, wobei der Substituent insbesondere Methyl, Methoxy, Halogen oder Trifluormethyl bedeutet, $R^1$ und $R^2$ gleich oder verschieden sind und jeweils Methyl, Ethyl, Isopropyl, Allyl, Cyclohexyl, Cyclopentyl, Methoxy, Brom oder Chlor bedeuten,
$R^3$ Wasserstoff oder Methyl

X 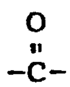 , 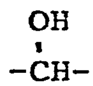 , 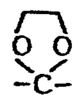 , oder 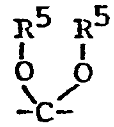 bedeutet,

wobei $R^5$ vorzugsweise Methyl oder Ethyl bedeutet und
Z eine gegebenenfalls mono-substituierte Phenylgruppe bedeutet, wobei der Substituent insbesondere Halogen, Methoxy oder Trifluormethyl bedeutet,
oder einen Rest der Formel Z (a), in der $R^6$ Methyl, Ethyl, Propyl, Brom, Chlor oder Halogenmethyl bedeutet.

Stärker bevorzugt sind Verbindungen der Formel I, in denen D Pyrazol-1-yl oder Imidazol-1-yl bedeutet, $R^1$ und $R^2$ gleich oder verschieden sind und jeweils Methyl, Ethyl, Isopropyl, Allyl, Methoxy oder Chlor bedeuten,
$R^3$ Wasserstoff, X 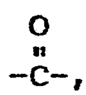, 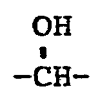 oder 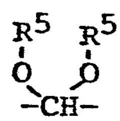 bedeutet, wobei $R^5$ vorzugsweise Methyl oder Ethyl ist,
Z einen Rest der Formel Z (a) bedeutet, in der $R^6$ Methyl, Chlormethyl oder Fluormethyl bedeutet.

Noch stärker bevorzugt sind Verbindungen der Formel I, in denen D Imidazol-1-yl, $R^1$ und $R^2$ gleich oder verschieden sind und jeweils Methyl, Ethyl oder Chlor bedeuten,
$R^3$ Wasserstoff, X 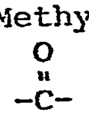 oder 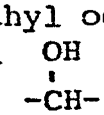 und Z tert.-Butyl bedeutet.

Die Erfindung betrifft ferner ein Verfahren zur Herstellung von Verbindungen der Formel I und von deren Salzen, das dadurch gekennzeichnet ist, daß man eine Verbindung der

Formel II

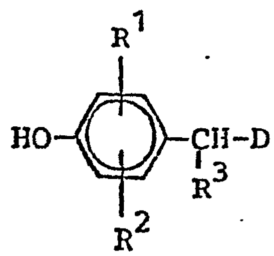

II

in der $R^1$, $R^2$, $R^3$ und D die zu Formel I angegebenen Bedeutungen haben, mit einer Verbindung der Formel III

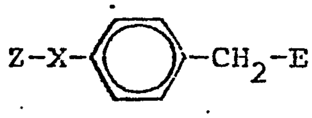

III

in der X und Z die zu Formel I angegebenen Bedeutungen haben und E Halogen, vorzugsweise Brom oder Chlor bedeutet, umsetzt, gegebenenfalls erhaltene Verbindungen, worin X die 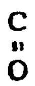 Gruppe bedeutet, zu Verbindungen der Formel I worin X = 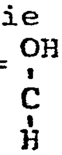 bedeutet, reduziert oder zu Verbindungen worin X = 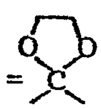 oder 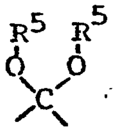 in an sich bekannter Weise ketalisiert, und die Reaktionsprodukte gegebenenfalls mit Säuren in physiologisch verträgliche Säureadditionssalze überführt.

Die vorgenannte Umsetzung wird in einem Temperaturbereich von 30 bis 150°C, vorzugsweise 40 bis 100°C, in Anwesenheit einer Base und in einem inerten organischen Lösungsmittel, wie N,N-Dimethylformamid, N,N-Dimethylacetamid, Dimethylsulfoxid, N-Methylpyrrolidon-2, Dioxan, Tetrahydrofuran, 4-Methyl-2-pentanon, Methanol, Ethanol, Isopropylalkohol, sek.-Butanol, Cyclohexanol, Propanol, Butanol, Pentanol, tert.-Butylalkohol, Methylglykol, Methylenchlorid, Tetrachlorkohlenstoff oder einem aromatischen Kohlenwasserstoff wie Benzol, Chlorbenzol, Toluol oder Xylol oder in Wasser durchgeführt. Es können auch Gemische der beispielhaft genannten Lösungsmittel verwendet werden.

Geeignete Basen sind beispielsweise Alkalimetall- oder Erdalkalimetall-carbonate, -hydrogencarbonate, -hydroxide, -alkoholate oder -hydride wie z.B. Natriumcarbonat, Natriumhydrogencarbonat, Kaliumcarbonat, Natriumhydroxid, Natriummethylat oder Natriumhydrid, oder organische Basen z.B. tertiäre Amine wie Triethylamin, Tributylamin, Ethylmorpholin oder Pyridin, Dimethylaminopyridin, Chinolin oder 1,5-Diazabicyclo[5,4,0]undec-5-en(1,8-7) (DBU), 1-H-Imidazol oder 1-H-1,2,4-Triazol.

Die Umsetzung von Verbindungen der Formel II mit Verbindungen der Formel III erfolgt ebenso z.B. unter den Bedingungen einer Phasentransferreaktion, indem man die Reaktionspartner in einem der vorstehenden Lösungsmittel, unter kräftigem Rühren in Gegenwart eines Phasentransferkatalysators und entweder einem gepulverten Alkalihydroxid, wie z.B. Natrium- oder Kaliumhydroxid oder einer konzentrierten wäßrigen Lösung derselben, vorzugsweise in einem Temperaturbereich von 20 bis 120°C aufeinander einwirken läßt.

Geeignete Phasentransferkatalysatoren sind z.B. Trialkylbenzylammonium- oder Tetraalkylammonium-halogenide, -hydroxide, -hydrogensulfate mit vorzugsweise 1 bis 12 C-Atomen im Alkylrest oder Kronenether, wie z.B. 12-Crown-4, 15-Crown-5, 18-Crown-6 oder Dibenzo-18-crown-6.

Solche Verbindungen der Formel I in denen X $-\overset{OH}{CH}-$ bedeutet, werden zweckmäßig hergestellt, indem man eine Verbindung der Formel I, in der X eine Carbonylgruppe bedeutet, mit einem geeigneten Reduktionsmittel umsetzt.

Die vorgenannte Reduktion wird zweckmäßig in einem Temperaturbereich von 0 bis 100°C, gegebenenfalls in einem Autoklaven unter einem Wasserstoffdruck von bis zu 100 atm., in einem inerten Lösungsmittel durchgeführt, wie sie vorstehend bei der Umsetzung von Verbindungen der Formel II mit III genannt sind. Geeignete Reduktionsmittel sind z.B. komplexe

Metallhydride wie $LiAlH_4$, $NaCNBH_4$, $NaBH_4$, $LiBH_4$, Diisobutylaluminiumhydrid, Alkoholate des Aluminiums mit sekundären Alkoholen, insbesondere mit Isopropanol, sek.-Butanol und Cyclohexanol oder Metallkatalysatoren wie z.B. Pd/Kohle oder Raney-Nickel.

Herstellung der Ausgangsstoffe

Herstellung von Verbindungen der Formel II:
Die Ausgangsverbindungen der Formel II sind entweder bekannt oder können nach an sich bekannten Verfahren erhalten werden, z.B. durch Umsetzung eines entsprechenden Phenols mit einem Aldehyd der Formel $R^3$-CH=O, in der $R^3$ Wasserstoff oder $C_1$-$C_4$-Alkyl bedeutet und einem Amin der Formel H-D, in der D die zu Formel I angegebenen Bedeutungen hat (vergl. dazu H. Hellmann und G. Opitz, "$\alpha$-Aminoalkylierung", Verlag Chemie Weinheim (1969)).

Ein weiteres Verfahren besteht darin, daß man bevorzugt entsprechende Dimethyl- oder Diethylamino-Phenol-Mannich-Basen mit einer Aminoverbindung der Formel H-D, in der D die vorstehende Bedeutung hat, in Substanz oder vorzugsweise in einem inerten organischen Lösungsmittel auf 50 - 180°C erhitzt (vgl. dazu DE-OS 30 39 087).

Diejenigen Verbindungen der Formel II, in denen $R^3$ $C_1$-$C_4$-Alkyl bedeutet und $R^1$, $R^2$ und D die zu Formel I angegebenen Bedeutungen haben, werden vorzugsweise analog zu den in Eur.J.Med.Chem. 1979, S. 227-245 beschriebenen Methoden hergestellt.

Herstellung von Verbindungen der Formel III:
Die Verbindungen der Formel III, in denen X eine Carbonylgruppe bedeutet, sind entweder bekannt oder können nach an sich bekannten Verfahren erhalten werden. So werden Verbindungen der Formel III, in denen X eine Carbonylgruppe und Z ein Rest der Formel Z (a) bedeutet,*gemäß dem in DE-OS 25 14 960 angegebenen Verfahren hergestellt. Die übrigen

*vorzugsweise

Verbindungen der Formel III, in denen X eine Carbonylgruppe bedeutet, werden*durch Halogenierung der entsprechenden Methylbenzophenone hergestellt. Verbindungen der Formel III worin X die 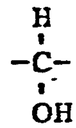 , 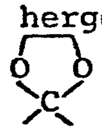 oder 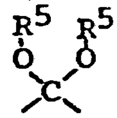 Gruppe darstellt, können prinzipiell durch Reduktion oder Ketalisierung der entsprechenden Carbonylverbindung hergestellt werden.

*zweckmäßig

Verbindungen der Formel III'

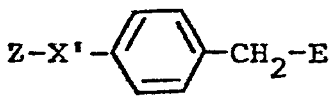 III'

worin

Z die zu Formel I und E die zu Formel III angegebenen Bedeutungen haben und X' 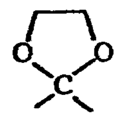 oder 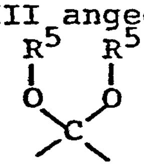 wobei $R^5$ $C_1$-$C_4$-Alkyl ist, bedeutet, sind neu.

Die Erfindung betrifft daher auch Verbindungen der Formel III' sowie Verfahren zu ihrer Herstellung. Die Verfahren sind dadurch gekennzeichnet, daß man

a) eine Verbindung der Formel IV

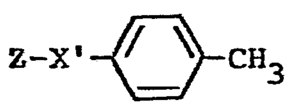 IV

worin Z die zu Formel I und X' die zu Formel III' angegebenen Bedeutungen haben, in an sich bekannter Weise halogeniert oder

b) eine Verbindung der Formel V

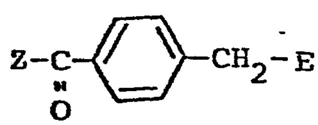 V

worin Z die zu Formel I und E die zu Formel III angegebenen Bedeutungen haben, mit Ethylenglykol oder einem Alkohol $R^5$-OH, wobei $R^5$ $C_1$-$C_4$-Alkyl bedeutet, umsetzt.

Die Halogenierung nach Verfahren a) erfolgt vorzugsweise mit Brom oder Chlor, in einem inerten Lösungsmittel oder in Substanz, gegebenenfalls unter Bestrahlung, in einem Temperaturbereich von 30 bis 170°C (vgl. z.B. CA 70, 106 212 f, 1969). Bevorzugt werden Verbindungen der Formel III' hergestellt, indem man Verbindungen der Formel IV mit N-Chlorsuccinimid oder N-Bromsuccinimid, in einem inerten Lösungsmittel, in Gegenwart eines Radikalstarters wie z.B. Benzoylperoxid oder Azo-bis-isobutyronitril, in einem Temperaturbereich von 40-100°C umsetzt.
Als Lösungsmittel für die vorstehend genannten Umsetzungen werden diejenigen verwendet, die für die Umsetzung von Verbindungen der Formel II mit III genannt sind.

Die Herstellung von Verbindungen der Formel III' gemäß Verfahren b durch Reaktion von Verbindungen der Formel V mit Ethylenglykol erfolgt in analoger Weise wie in Synthesis, 1974 (I) S. 23 beschrieben und mit einem Alkohol $R^5$-OH in analoger Weise wie von O. Exner in Collect 16/17 S. 265 (1951) beschrieben.

Die Verbindungen der Formel III' können z.B. zur Herstellung von Verbindungen der Formel I, worin X die Bedeutung von X' hat, verwendet werden.

Es ist zweckmäßig in Verbindungen der Formel III, worin X eine Carbonylgruppe bedeutet, die Carbonylgruppe erst nach der Reaktion mit Verbindungen der Formel II zur $-\overset{OH}{\underset{H}{C}}-$ Gruppe zu reduzieren.

Zur Herstellung von physiologisch verträglichen Säureadditionssalzen kommen vorzugsweise folgende Säuren in Frage: Halogenwasserstoffsäuren, wie z.B. Chlorwasserstoff- und Bromwasserstoff-säure, sowie Salpetersäure, ferner Phosphorsäure oder Schwefelsäure. Bevorzugte organische Säuren sind mono- und bifunktionelle Carbonsäuren und Hydroxycarbonsäuren wie z.B. Essigsäure, Maleinsäure, Oxalsäure, Bernsteinsäure, Fumarsäure, Weinsäure, Zitronensäure, Salicylsäure, Sorbinsäure, Milchsäure sowie Sulfonsäuren wie z.B. p-Toluolsulfonsäure, Methyl- und Phenylsulfonsäure sowie 1,5-Naphthalindisulfonsäure.

Die Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure oder Salpetersäure, erhalten werden und in bekannter Weise, z.B. durch Abfiltrieren, isoliert und gegebenenfalls durch Waschen oder Umkristallisation mit einem inerten organischen Lösungsmittel gereinigt werden.

Aus der Formel I ist ersichtlich, daß die erfindungsgemäßen Verbindungen bis zu zwei asymmetrische Kohlenstoffatome aufweisen können. Ein Asymmetriezentrum ist vorhanden, wenn $R^3$ $C_1$-$C_4$-Alkyl und X nicht $-\overset{OH}{CH}-$ bedeutet oder wenn $R^3$ Wasserstoff und X $-\overset{OH}{CH}-$ bedeutet. Zwei Asymmetriezentren sind vorhanden, wenn X $-\overset{OH}{CH}-$ und $R^3$ $C_1$-$C_4$-Alkyl bedeutet.

Die diastereomeren Racemate der Verbindungen der Formel I (für den Fall zweier Asymmetriezentren) können in üblicher Weise, z.B. durch selektive Kristallisation oder Chromatographie getrennt werden. Racemate können ihrerseits in üblicher Weise z.B. nach den in E.L. Eliel, Stereochemie der Kohlenwasserstoffverbindungen, 1966. Verlag Chemie Weinheim, beschriebenen Methoden in ihre Antipoden getrennt werden.

Die erfindungsgemäßen Verbindungen der Formel I besitzen wertvolle pharmakologische Eigenschaften, insbesondere zeigen sie eine sehr starke und günstige Beeinflussung der Serumlipoproteine. Die Erfindung betrifft daher auch pharmazeutische Präparate auf Basis dieser Verbindungen sowie ihre Verwendung als Arzneimittel, insbesondere zur Beeinflussung der Serumlipoproteine.

Es ist allgemein anerkannt, daß für die Entstehung arteriosklerotischer Gefäßveränderungen, insbesondere der coronaren Herzkrankheit, Hyperlipoproteinämien einen wesentlichen Risikofaktor darstellen. Für die Prophylaxe und die Regression von arteriosklerotischen Veränderungen kommt daher der Senkung erhöhter Serum-Lipoproteine eine außerordentliche Bedeutung zu. Hierbei kommt es aber auf ganz bestimmte Klassen von Serum-Lipoproteinen an, da die low density (LDL) und very low density-Lipoproteine (VLDL) einen atherogenen Risikofaktor darstellen, während die high density-Lipoproteine (HDL) eine Schutzfunktion gegenüber arteriosklerotischer Gefäßveränderung ausüben. Hypolipidämika sollen demnach VLDL-Cholesterin und LDL-Cholesterin im Serum erniedrigen, dabei aber die HDL-Cholesterin-Konzentration nach Möglichkeit unbeeinflußt lassen oder sogar erhöhen, sodaß der antiarteriogene Index $\frac{HDL}{LDL}$ zunimmt.

Die erfindungsgemäßen Verbindungen haben wertvolle therapeutische Eigenschaften. So erniedrigen sie vor allem die Konzentration von LDL und VLDL, die HDL-Fraktion aber kaum, in vielen Fällen wird diese Fraktion sogar vermehrt. Diese Verbindungen stellen daher einen wesentlichen Fortschritt gegenüber der Vergleichsverbindung Clofibrat dar, wie aus den nachfolgend beschriebenen Daten ersichtlich ist. Sie können daher zur Prophylaxe und Regression von arteriosklerotischen Veränderungen herangezogen werden, indem sie einen kausalen Risikofaktor ausschalten. Hierzu zählen nicht nur die primären Hyperlidämien, sondern auch gewisse sekundäre Hyperlipidämien wie sie z.B. beim Diabetes vorkommen. Das relative Lebergewicht wird durch die Verbindungen in wirksamer Dosis nicht oder nur geringfügig vermehrt, während das als hypolipidämischer Standard verwendete Clofibrat zu einer starken Erhöhung des relativen Lebergewichts führt. Die mit einer hypolipidämischen Wirkung verbundene unerwünschte Beeinflussung der Leber zeigt sich

auch in sehr starkem Maße bei der in J.Med.Chem. 18 (1975) S. 833-836 beschriebenen Vergleichsverbindung 1-(4-Methylbenzyl)-imidazol. Mit der in den hohen Dosen starken hypolipidämischen Wirkung der Vergleichsverbindung die gleichermaßen atherogene (VLDL und/oder LDL) und antiatherogene (HDL) Lipoproteinklassen betrifft, ist eine massive Hepatomegalie mit massiven Neutralfett- und Cholesterineinlagerungen ("Fettleber") verbunden. Die erfindungsgemäßen Verbindungen zeigen dieses unerwünschte Verhalten nicht.

Die Wirkung der angeführten Verbindungen auf die Serum-Lipoproteine wurde an männlichen Wistar-Ratten untersucht, die 7 Tage per Schlundsonde mit den angeführten in Polyäthylenglykol 400 suspendierten Verbindungen behandelt wurden. Außerdem wurde eine Kontrollgruppe, die nur das Lösungsmittel Polyäthylenglykol 400 erhielt, mitgeführt, sowie bei den meisten Versuchen eine Ratten-Gruppe mit dem Standardhypolipidämikum Clofibrat. Pro Gruppe wurden in der Regel 10 Tiere eingesetzt, denen am Ende der Behandlung das Blut nach leichter Äthernarkose aus dem Orbitalplexus entnommen wurde. Aus dem gewonnenen Ratten-Serum wurden die Serum-Lipoproteine in der präparativen Ultrazentrifuge in folgende Dichteklassen getrennt:

VLDL $<$ 1,006; LDL 1,006 bis 1,04; HDL 1,04 bis 1,21

Da nämlich im Gegensatz zum Menschen die Serumlipoproteine der Ratte etwa 4/5 HDL-Cholesterin und nur 1/5 LDL-Cholesterin enthalten und nur sehr geringe Mengen von VLDL (beim Menschen umgekehrt etwa 2/3 LDL und VLDL und nur 1/3 bis 1/4 HDL), macht die Beurteilung einer hypolipidämischen Wirkung an der Ratte unbedingt eine Fraktionierung des Rattenserums in Lipoproteinklassen erforderlich. Eine bloße Erniedrigung des Serum-Total-cholesteringehaltes an der Ratte würde nämlich nur die unerwünschte Erniedrigung der bei der Ratte vorwiegenden antiatherogenen HDL-Klasse anzeigen. Eine erwünschte Erniedrigung von LDL bei gleichzeitiger erwünschter Vermehrung von HDL hätte aber keine

(wesentliche) Auswirkung auf den Totalcholesteringehalt des Ratten-Serums.

Aus den in der Ultrazentrifuge isolierten Lipoprotein-Fraktionen wurde das darin enthaltene Cholesterin vollenzymatisch nach der CHOD-PAP-Methode mittels der Testkombination von Boehringer-Mannheim bestimmt und die Werte in µg/ml Serum umgerechnet. In der angeführten Tabelle ist die Veränderung des Lipoprotein-Cholesterins in der behandelten Gruppe gegenüber einer unter gleichen Bedingungen mitgeführten Kontrollgruppe angegeben. Wie aus der Tabelle ersichtlich, bewirkt Clofibrat eine stärkere Verminderung der HDL- als der LDL-Fraktion, während die neuen Verbindungen eine starke selektiv senkende Wirkung auf die atherogenen Lipoproteinfraktionen (VLDL und LDL) ausüben und die schützende HDL-Fraktion im wesentlichen unbeeinflußt lassen oder sogar vermehren.

0104572

Veränderung in % der Lipoproteine im Ratten-Serum nach siebentägiger oraler Applikation der Verbindungen

| Beispiel Nr. | Dosierung mg/kg / Tag | Cholesterolveränderung in %, bezogen auf die Kontrollgruppe | | | | | % Veränderung des relativen Lebergewichtes |
|---|---|---|---|---|---|---|---|
| | | im Serum | in den Serum-Lipoprotein-Fraktionen | | | | |
| | | | VLDL | LDL | HDL | HDL/LDL | |
| 1 | 10 | -17 | 0 | -52 | -5 | 1.97 | +3 |
| | 3 | -12 | 0 | -42 | +3 | 1.77 | +9 |
| | 1 | - 4 | 0 | -40 | +8 | 1.81 | +4 |
| 3 | 10 | - 8 | -58 | -45 | +2 | 1.86 | +6 |
| | 3 | - 2 | +17 | -36 | +2 | 1.60 | -4 |
| | 1 | + 2 | +30 | -33 | +5 | 1.58 | -2 |
| 1.2 | 30 | - 7 | -31 | -39 | +9 | 1.82 | +20 |
| 1.6 | 10 | - 3 | - 2 | -32 | +7 | 1.58 | +16 |
| 1.7 | 20 | -18 | - 6 | -43 | -7 | 1.62 | + 9 |
| 1.8 | 20 | -19 | -19 | -43 | -4 | 1.69 | + 1 |
| 1.16 | 10 | -15 | -24 | -52 | -1 | 2.05 | + 6 |
| | 3 | - 8 | -32 | -32 | -11 | 1.32 | + 7 |
| 1.19 | 10 | -11 | -35 | -29 | - 1 | 1.40 | + 1 |
| 1.21 | 30 | 0 | -62 | -35 | + 4 | 1.59 | - 7 |
| 1.27 | 5 | -11 | - 5 | -51 | - 3 | 1.70 | - 8 |
| 1.28 | 30 | + 3 | - 9 | -31 | +21 | 1.76 | 0 |
| 1.29 | 20 | - 8 | -35 | -32 | + 4 | 1.53 | + 7 |
| 1.46 | 10 | - 5 | -29 | -35 | + 4 | 1.61 | + 4 |
| 1.52 | 30 | + 1 | -36 | -23 | + 4 | 1.34 | +11 |
| 5 | 10 | -23 | -46 | -59 | - 6 | 2.24 | +12 |
| | 3 | + 4 | -10 | -47 | +18 | 2.25 | +13 |
| | 1 | - 7 | -13 | -39 | - 6 | 1.54 | + 2 |
| 2.1 | 10 | -22 | -54 | -66 | - 3 | 2.85 | + 7 |
| | 1 | - 6 | +17 | -53 | +17 | 2.47 | +11 |
| 2.2 | 30 | - 4 | -34 | -49 | +14 | 2.26 | +16 |
| 2.4 | 30 | -12 | -50 | -38 | - 7 | 1.51 | +23 |
| Clofibrat | 100 | -29 | -48 | -18 | -34 | 0.70 | +33 |
| 1-(4-Methylbenzyl)-imidazol | 30 | -44 | -100 | -90 | -24 | 7.41 | +49 |
| | 10 | +16 | -23 | -3 | +22 | 1.25 | + 2 |
| | 3 | +11 | +10 | -5 | +18 | 1.24 | 0 |

Als therapeutische Zubereitung der Verbindungen der Formel I kommen vor allem Tabletten, Dragees, Kapseln, Suppositorien und Säfte in Frage. Die neuen Verbindungen können dabei entweder allein oder mit pharmakologisch annehmbaren Trägern vermischt, angewandt werden. Eine orale Anwendungsform wird bevorzugt. Zu diesem Zweck werden die aktiven Verbindungen vorzugsweise mit an sich bekannten Substanzen vermischt und durch an sich bekannte Methoden in geeignete Darreichungsformen gebracht, wie Tabletten, Steckkapseln, wässrige oder ölige Suspensionen oder wässrige oder ölige Lösungen. Als inerte Träger können z.B. Magnesiumkarbonat, Milchzucker oder Maisstärke unter Zusatz anderer Stoffe wie z.B. Magnesiumstearat verwendet werden. Dabei kann die Zubereitung sowohl als Trocken- oder Feuchtgranulat erfolgen. Als ölige Trägerstoffe oder Lösungsmittel kommen besonders pflanzliche und tierische Öle in Betracht wie z.B. Sonnenblumenöl oder Lebertran. Als tägliche Dosis kommen etwa 50 mg bis 3 g, vorzugsweise 100 bis 500 mg in Betracht. Eine Dosierungseinheit enthält vorzugsweise 100 bis 300 mg.

Die Zubereitungen können bei der Behandlung von Lipidstoffwechselstörungen außer den üblichen Füll- und Trägerstoffen noch ein Antihypertensivum, wie beispielsweise ein Saluratikum, Reserpin, Hydralazin, Guanethidin, α-Methyldopa, Clonidin oder ein ß-Sympathikolytikum, eine antithrombotisch wirksame Verbindung, wie z.B. Acetylsalicylsäure, Sulfinpyrazon, Ticlopidin und Heparinoide, oder ein antihyperurikämisch wirksames Mittel, ein orales Antidiabetikum, ein Geriatrikum oder ein Mittel mit durchblutungssteigernder Wirkung enthalten.

0104572

Die Beispiele erläutern die Erfindung.

Beispiele für das Herstellverfahren nach Anspruch 2

## Beispiel 1

**4-/2-Chlor-4-(-imidazol-1-ylmethyl)-6-methylphenoxymethyl7-phenyl-tert.-butyl-keton**

Zur Mischung aus 4,05 g = 20 mmol 2-Chlor-6-methyl-4-(imidazol-1-ylmethyl)-phenol, 30 ml Toluol, 5 ml 50 %-iger Natronlauge und 0,3 g Tetrabutylammoniumbromid tropft man unter intensiver Rührung bei 90 °C die Lösung von 5,1 g = 20 mmol 4-Brommethyl-phenyl-tert.-butyl-keton in 20 ml Toluol innerhalb 30 min zu, rührt bei 90 °C 4 h nach und läßt abkühlen. Anschließend werden 20 ml 6 n Natronlauge zugegeben, kurz durchgerührt, die organische Phase abgetrennt, mit Wasser gewaschen, über $Na_2SO_4$ getrocknet und im Vakuum abgezogen. Der erhaltene Rückstand (6,0 g) wird säulenchromatographisch an Kieselgel (Kieselgel S, Riedel-de Haen) mit einer Mischung aus Methylenchlorid: Ethanol, 20 : 1 als Laufmittel gereinigt. Das dabei anfallende Oel wird in Ether/Diisopropylether aufgenommen, der entstehende Niederschlag abgesaugt. Man erhält 4 g vom Fp.: 100 °C (50 % d.Th.).

Analyse:

| | | | | |
|---|---|---|---|---|
| $C_{23}H_{25}ClN_2O_2$ | ber.: | C 69,60 | gef.: | C 69,0 |
| MG: 396,92 | | H 6,35 | | H 6,4 |
| | | N 7,06 | | N 7,1 |

Bei gleicher Arbeitsweise wie vorstehend beschrieben können auch Benzol, Chlorbenzol und Xylol als Lösungsmittel verwendet werden. Im Falle von Methylenchlorid als Lösungsmittel arbeitet man bei Rückflußtemperatur.

Beispiel 2

4-[2,5-Dimethyl-4-(imidazol-1-ylmethyl)-phenoxymethyl]-phenyl-tert.-butyl-keton

a) Herstellung der freien Base

Zur Suspension aus 5,52 g = 40 mmol Kaliumcarbonat und 4,04 g = 20 mmol 2,5-Dimethyl-4-(imidazol-1-ylmethyl)-phenol in 20 ml Dimethylformamid tropft man unter Rühren bei 80 °C die Lösung von 5,1 g = 20 mmol 4-Brommethylphenyl-tert.-butylketon in 10 ml Dimethylformamid und rührt 5 h bei 80 °C nach. Die abgekühlte Lösung wird mit 200 ml Wasser verdünnt, mit Methylenchlorid ausgeschüttelt, *) Der erhaltene Rückstand (7,5 g) wird säulenchromatographisch gereinigt, wie im Beispiel 1 beschrieben. Man erhält 5,3 g (70 % d.Th.).

Analyse:

| | | | |
|---|---|---|---|
| $C_{24}H_{28}N_2O_2$ | ber.: C 76,56 | gef.: C 76,3 | |
| MG: 376,50 | H 7,50 | H 7,4 | |
| | N 7,44 | N 7,1 | |

*) die organische Phase wird über Natriumsulfat getrocknet und anschließend entfernt man das Lösungsmittel im Vakuum.

b) Herstellung des Hydrochlorides

Zur Lösung von 3,76 g = 10 mmol der vorstehend beschriebenen Verbindung in 5 ml Methanol tropft man unter Rühren 11 ml 1 n methanolischer HCl, zieht anschließend i.V. das Lösungsmittel ab, nimmt den Rückstand in 20 ml Essigester auf und saugt anschließend das Kristallisat ab. Man erhält 3,8 g vom Fp.: 177 - 178 °C (92 % d.Th.) 4-/2,5-Dimethyl-4-(imidazol-1-ylmethyl)-phenoxymethyl7-phenyl-tert.-butylketon als Hydrochlorid.

Analyse:

| | | | |
|---|---|---|---|
| $C_{24}H_{29}ClN_2O_2$ | ber.: C 69,80 | | gef.: C 69,8 |
| MG: 412,96 | H 7,08 | | H 7,0 |
| | N 6,78 | | N 6,6 |

Beispiel 3

4-/2,6-Dimethyl-4-(imidazol-1-ylmethyl)-phenoxymethyl7-phenyl-tert.-butyl-keton

a) Herstellung der freien Base

Zur Suspension aus 0,6 g = 20 mmol NaH (80 %-ige Oeldispersion) in 30 ml abs. Dimethylformamid gibt man unter Rühren und Kühlen mit einem Eisbad 4,04 g = 20 mmol 2,6-Dimethyl-4-(imidazol-1-ylmethyl)-phenol portionsweise so zu, daß die Temperatur 30 °C nicht übersteigt, rührt

nach bis die Wasserstoffentwicklung beendet ist und tropft anschließend bei 0 °C die Lösung von 5,1 g = 20 mmol 4-Brommethylphenyl-tert.-butylketon in 10 ml abs. Dimethylformamid zu. Man läßt auf Raumtemperatur aufwärmen und rührt 6 h bei 80 °C nach, zieht i.V. das Lösungsmittel ab und arbeitet *) mit $CH_2Cl_2$/Wasser auf. Der Rückstand (7,3 g) wird säulenchromatographisch gereinigt, wie im Beispiel 1 beschrieben. Man erhält 4,9 g vom Fp. 50-52 °C (65 % d.Th.).

*) wie in Beispiel 2 angegeben

Analyse:

| | | | | |
|---|---|---|---|---|
| $C_{24}H_{28}N_2O_2$ | ber. | C 76,56 | gef.: | C 76,4 |
| | | H 7,50 | | H 7,5 |
| | | N 7,44 | | N 7,4 |

Bei gleicher Arbeitsweise wie vorstehend beschrieben können auch Dimethylsulfoxid, Dimethylacetamid, oder N-Methylpyrrolidon-2, sowie Gemische dieser Lösungsmittel mit z.B. Tetrahydrofuran, Dioxan, Dimethoxyethan oder Toluol verwendet werden.

b) Herstellung des Hydrochlorides

In die Lösung von 11,3 g = 30 mmol der vorstehend beschriebenen Verbindung in 100 ml Essigester leitet man bei 20 °C bis zur Sättigung HCl ein, zieht anschließend i.V. das Lösungsmittel ab und kristallisiert den Rückstand aus Methylisobutylketon um. Man erhält 10,8 g vom Fp.: 170 °C (87 % d.Th.) 4-[2,6-Dimethyl-4-(imidazol-1-ylmethyl)-phenoxymethyl]-phenyl-tert.-butyl-keton als Hydrochlorid.

Analyse:

| | | | |
|---|---|---|---|
| $C_{24}H_{29}ClN_2O_2$ | ber.: | C 69,81 | gef.: 69,8 |
| MG: 412,95 | | H 7,08 | 7,0 |
| | | N 6,78 | 6,8 |

Beispiel 4

Gemäß Beispiel 3, jedoch unter Verwendung äquivalenter Mengen der entsprechenden Verbindungen der Formeln II und III können die folgenden, in Tabelle 1 aufgeführten, Verbindungen der Formel I hergestellt werden.

Tabelle 1

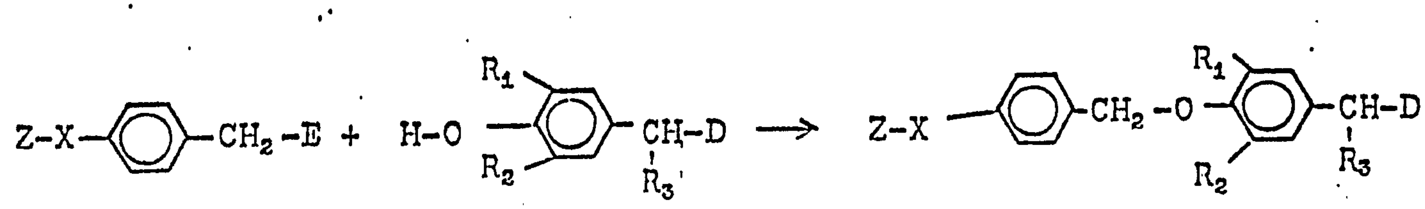

III II I

| Verbdgs. Nr. | Z | X | $R_1$ | $R_2$ | $R_3$ | D | Fp.: [°C] | Analyse: ber.: | gef.: |
|---|---|---|---|---|---|---|---|---|---|
| 1.1 | tert.-butyl | $-\overset{O}{\overset{\|}{C}}-$ | H | H | H | 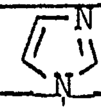 | 92-94 | C 75,83<br>H 6,94<br>N 8,04 | C 75,5<br>H 7,0<br>N 8,0 |
| 1.2 | tert.-butyl | $-\overset{O}{\overset{\|}{C}}-$ | $CH_3$ | allyl | H | 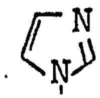 | Oel | C 77,56<br>H 7,53<br>N 6,96 | C 76,0<br>H 7,4<br>N 7,0 |
| 1.3 | tert.-butyl | $-\overset{O}{\overset{\|}{C}}-$ | iso-propyl | iso-propyl | H | 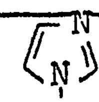 | 125-126 | C 77,74<br>H 8,39<br>N 6,48 | C 77,5<br>H 8,4<br>N 6,3 |
| 1.4 | tert.-butyl | $-\overset{O}{\overset{\|}{C}}-$ | tert.-butyl | tert.-butyl | H | 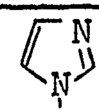 | | | |
| 1.5 | tert.-butyl | $-\overset{O}{\overset{\|}{C}}-$ | $C_2H_5$ | $C_2H_5$ | H | 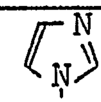 | Oel | C 77,20<br>H 7,97<br>N 6,93 | C 77,1<br>H 8,1<br>N 7,2 |
| 1.6 | tert.-butyl | $-\overset{O}{\overset{\|}{C}}-$ | Cl | Cl | H | 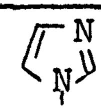 | Oel | C 63,32<br>H 5,31<br>N 6,71 | C 63,0<br>H 5,5<br>N 6,4 |
| 1.7 | tert.-butyl | $-\overset{O}{\overset{\|}{C}}-$ | Cl | allyl | H | 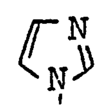 | Oel | C 70,98<br>H 6,43<br>N 6,62 | C 70,5<br>H 6,5<br>N 6,6 |

| Verbdgs.-Nr. | Z | X | $R_1$ | $R_2$ | $R_3$ | D | Fp.: [°C] | ber.: | gef.: |
|---|---|---|---|---|---|---|---|---|---|
| 1.8 | tert.-butyl | O<br>‖<br>-C- | $-OCH_3$ | $-OCH_3$ | H | 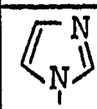 | Wachs | C 70,57<br>H 6,91<br>N 6,86 | C 70,2<br>H 6,7<br>N 6,8 |
| 1.9 | tert.-butyl | O<br>‖<br>-C- | $CH_3$ | $CH_3$ | $CH_3$ | 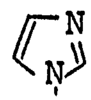 | Oel | C 76,89<br>H 7,74<br>N 7,17 | C 76,90<br>H 7,60<br>N 7,0 |
| 1.10 | tert.-butyl | O<br>‖<br>-C- | $CH_3$ | $CH_3$ | $C_2H_5$ | 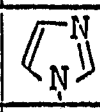 | | | |
| 1.11 | isopropyl | O<br>‖<br>-C- | $CH_3$ | $CH_3$ | H | 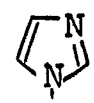 | Oel | C 76,21<br>H 7,23<br>N 7,73 | C 76,3<br>H 7,3<br>N 7,5 |
| 1.12 | $CH_3$<br>\|<br>$-C-CH_2-CH_3$<br>\|<br>$CH_3$ | O<br>‖<br>-C- | $CH_3$ | $CH_3$ | H | 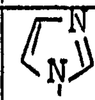 | Oel | C 76,89<br>H 7,74<br>N 7,17 | C 76,7<br>H 7,7<br>N 7,0 |
| 1.13 | $CH_3$<br>\|<br>$-C-(CH_2)_2-CH_3$<br>\|<br>$CH_3$ | O<br>‖<br>-C- | $CH_3$ | $CH_3$ | H | 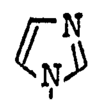 | | | |
| 1.14 | Br<br>\|<br>$-C-(CH_3)_2$ | O<br>‖<br>-C- | $CH_3$ | $CH_3$ | H | 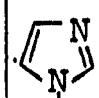 | | | |
| 1.15 | Cl<br>\|<br>$-C-(CH_3)_2$ | O<br>‖<br>-C- | $CH_3$ | $CH_3$ | H | 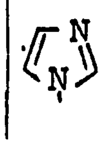 | | | |

0104572

| Verbdgs.-Nr. | Z | X | $R_1$ | $R_2$ | $R_3$ | D | Fp.: [°C] | ber.: | gef.: |
|---|---|---|---|---|---|---|---|---|---|
| 1.16 | $-\overset{CH_3}{\underset{CH_3}{C}}-CH_2-Cl$ | $-\overset{O}{\overset{\|}{C}}-$ | $CH_3$ | $CH_3$ | H | 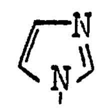 | Oel | C 70,15<br>H 6,62<br>N 6,82 | C 69,8<br>H 6,3<br>N 6,8 |
| 1.17 | $-\overset{CH_3}{\underset{CH_3}{C}}-CH_2-F$ | $-\overset{O}{\overset{\|}{C}}-$ | $CH_3$ | $CH_3$ | H | 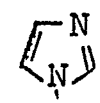 | | | |
| 1.18 | tert.-butyl | $-\overset{O}{\overset{\|}{C}}-$ | $CH_3$ | $CH_3$ | H | 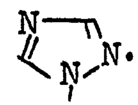 $\cdot HNO_3$ | Zers. 145 | C 62,71<br>H 6,41<br>N 12,72 | C 62,5<br>H 6,6<br>N 12,5 |
| 1.19 | tert.-butyl | $-\overset{O}{\overset{\|}{C}}-$ | Cl | $CH_3$ | H | 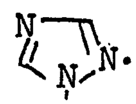 $\cdot HNO_3$ | Zers. 168-169 | C 57,33<br>H 5,47<br>N 12,15 | C 57,9<br>H 5,5<br>N 12,0 |
| 1.20 | tert.-butyl | $-\overset{O}{\overset{\|}{C}}-$ | $CH_3$ | $CH_3$ | H | 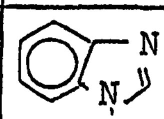 | Oel | C 78,84<br>H 7,09<br>N 6,57 | C 78,6<br>H 7,1<br>N 6,4 |
| 1.21 | tert.-butyl | $-\overset{O}{\overset{\|}{C}}-$ | $CH_3$ | $CH_3$ | H | 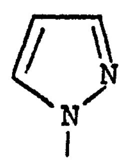 | Oel | C 76,56<br>H 7,5<br>N 7,44 | C 76,8<br>H 7,3<br>N 7,1 |

| Verbdgs.-Nr. | Z | X | $R_1$ | $R_2$ | $R_3$ | D | Fp.: [°C] | Analyse: ber.: | gef.: |
|---|---|---|---|---|---|---|---|---|---|
| 1.22 | tert.-butyl | $-\overset{O}{\overset{\|}{C}}-$ | $CH_3$ | $CH_3$ | H | 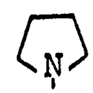 · HCl | 188-192 | C 72,18<br>H 8,24<br>N 3,37 | C 71,5<br>H 8,3<br>N 3,1 |
| 1.23 | tert.-butyl | $-\overset{O}{\overset{\|}{C}}-$ | $CH_3$ | $CH_3$ | H | 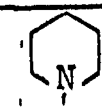 · HCl | 199-202 | C 72,62<br>H 8,44<br>N 3,26 | C 72,7<br>H 8,5<br>N 3,0 |
| 1.24 | tert.-butyl | $-\overset{O}{\overset{\|}{C}}-$ | $CH_3$ | $CH_3$ | H | 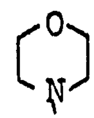 · HCl | 206-208 | C 69,51<br>H 7,93<br>N 3,24 | C 69,1<br>H 7,8<br>N 3,0 |
| 1.25 | tert.-butyl | $-\overset{O}{\overset{\|}{C}}-$ | $CH_3$ | $CH_3$ | H | 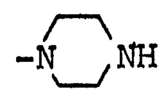 | | | |
| 1.26 | tert.-butyl | $-\overset{O}{\overset{\|}{C}}-$ | $CH_3$ | $CH_3$ | H | 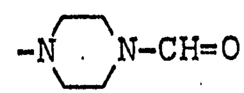 | 82-84 | C 73,90<br>H 8,11<br>N 6,63 | C 73,5<br>H 8,1<br>N 6,6 |
| 1.27 | tert.-butyl | $-\overset{O}{\overset{\|}{C}}-$ | $CH_3$ | $CH_3$ | H | 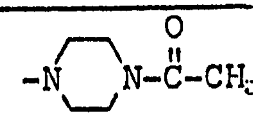 | 100-102 | C 74,28<br>H 8,31<br>N 6,42 | C 73,8<br>H 8,0<br>N 6,4 |

| Verbdgs. Nr. | Z | X | $R_1$ | $R_2$ | $R_3$ | D | Fp.: [°C] | Analyse: ber.: | gef.: |
|---|---|---|---|---|---|---|---|---|---|
| 1.28 | tert.-butyl | $-\overset{O}{\overset{\|}{C}}-$ | $CH_3$ | $CH_3$ | H | 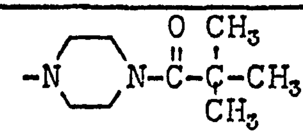 | 73-74 | C 75,23<br>H 8,84<br>N 5,85 | C 74,7<br>H 9,0<br>N 5,7 |
| 1.29 | tert.-butyl | $-\overset{O}{\overset{\|}{C}}-$ | $CH_3$ | $CH_3$ | H | 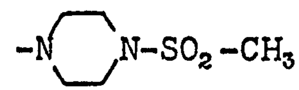<br>• $HNO_3$ | Zers. 138 | C 58,29<br>H 6,96<br>N 7,84 | C 57,9<br>H 7,2<br>N 7,7 |
| 1.30 | tert.-butyl | $-\overset{O}{\overset{\|}{C}}-$ | $CH_3$ | $CH_3$ | H | 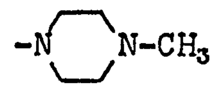 | Oel | C 76,43<br>H 8,88<br>N 6,86 | C 76,40<br>H 8,7<br>N 6,8 |
| 1.31 | tert.-butyl | $-\overset{O}{\overset{\|}{C}}-$ | $CH_3$ | $CH_3$ | H | 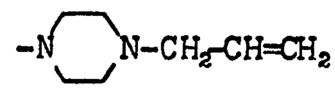 | | | |
| 1.32 | tert.-butyl | $-\overset{O}{\overset{\|}{C}}-$ | $CH_3$ | $CH_3$ | H | 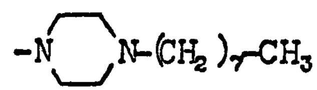 | | | |
| 1.33 | tert.-butyl | $-\overset{O}{\overset{\|}{C}}-$ | $CH_3$ | $CH_3$ | H | 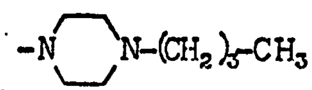 | | | |
| 1.34 | tert.-butyl | $-\overset{O}{\overset{\|}{C}}-$ | $CH_3$ | $CH_3$ | H | 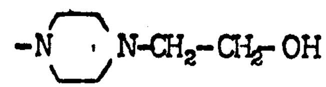 | | | |

| Verbdgs.-Nr. | Z | X | $R_1$ | $R_2$ | $R_3$ | D | Fp.: [°C] | Analyse: ber.: | gef.: |
|---|---|---|---|---|---|---|---|---|---|
| 1.35 | tert.-butyl | $-\overset{O}{\overset{\parallel}{C}}-$ | $CH_3$ | $CH_3$ | H | 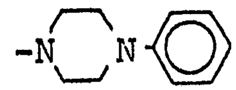 | | | |
| 1.36 | tert.-butyl | $-\overset{O}{\overset{\parallel}{C}}-$ | $CH_3$ | $CH_3$ | H | 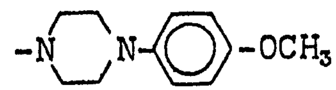 | Oel | C 76,77<br>H 8,05<br>N 5,60 | C 76,1<br>H 7,8<br>N 5,4 |
| 1.37 | tert.-butyl | $-\overset{O}{\overset{\parallel}{C}}-$ | $CH_3$ | $CH_3$ | H | 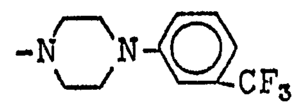 | | | |
| 1.38 | tert.-butyl | $-\overset{O}{\overset{\parallel}{C}}-$ | $CH_3$ | $CH_3$ | H | 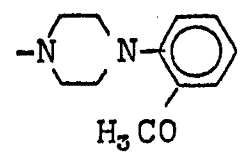 | | | |
| 1.39 | tert.-butyl | $-\overset{O}{\overset{\parallel}{C}}-$ | $CH_3$ | $CH_3$ | H | 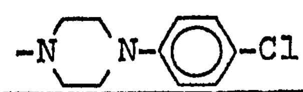 | | | |
| 1.40 | tert.-butyl | $-\overset{O}{\overset{\parallel}{C}}-$ | $CH_3$ | $CH_3$ | H | 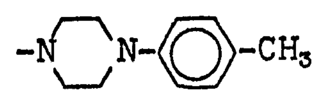 | | | |

| Verbdgs.-Nr. | Z | X | $R_1$ | $R_2$ | $R_3$ | D | Fp.: [°C] | Analyse: ber.: | gef.: |
|---|---|---|---|---|---|---|---|---|---|
| 1.41 | tert.-butyl | $-\overset{O}{\overset{\|}{C}}-$ | $CH_3$ | $CH_3$ | H | 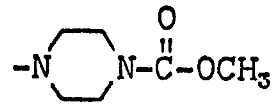 | | | |
| 1.42 | tert.-butyl | $-\overset{O}{\overset{\|}{C}}-$ | $CH_3$ | $CH_3$ | H | 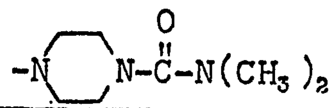 | | | |
| 1.43 | tert.-butyl | $-\overset{O}{\overset{\|}{C}}-$ | 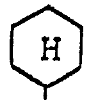 | 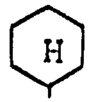 | H | 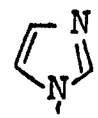 | | | |
| 1.44 | tert.-butyl | $-\overset{O}{\overset{\|}{C}}-$ | 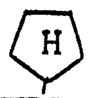 | 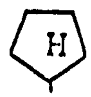 | H | 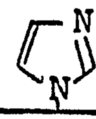 | | | |
| 1.45 | 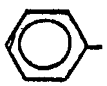 | $-\overset{O}{\overset{\|}{C}}-$ | $CH_3$ | $CH_3$ | H | 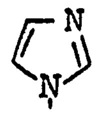 | 72-73 | C 78,76<br>H 6,10<br>N 7,06 | C 78,4<br>H 6,0<br>N 6,8 |
| 1.46 | 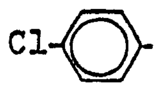 | $-\overset{O}{\overset{\|}{C}}-$ | $CH_3$ | $CH_3$ | H | 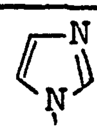 · $HNO_3$ | Zers. 71 ° | C 63,23<br>H 4,90<br>N 8,51 | C 63,0<br>H 4,9<br>N 8,4 |
| 1.47 | 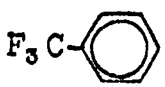 | $-\overset{O}{\overset{\|}{C}}-$ | $CH_3$ | $CH_3$ | H | 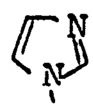 | 134-136 | C 69,82<br>H 4,99<br>N 6,05 | C 69,5<br>H 5,1<br>N 5,9 |

| Verbdgs.-Nr. | Z | X | $R_1$ | $R_2$ | $R_3$ | D | Fp.: [°C] | Analyse: ber.: | gef.: |
|---|---|---|---|---|---|---|---|---|---|
| 1.48 | 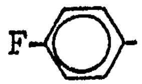 | 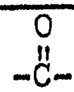 | $CH_3$ | $CH_3$ | H | 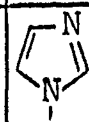 | 80-82 | C 75,34<br>H 5,59<br>N 6,76 | C 75,5<br>H 5,9<br>N 6,8 |
| 1.49 | 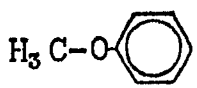 | 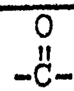 | $CH_3$ | $CH_3$ | H | 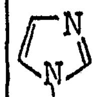 | | | |
| 1.50 | 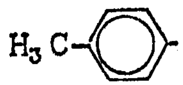 | 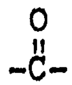 | $CH_3$ | $CH_3$ | H | 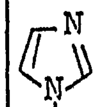 | | | |
| 1.51 | 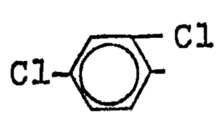 | 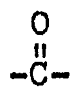 | $CH_3$ | $CH_3$ | H | 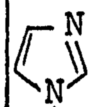 | | | |
| 1.52 | tert.-butyl | 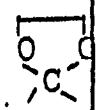 | $CH_3$ | $CH_3$ | H | 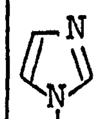 | 119-121 | C 74,26<br>H 7,67<br>N 6,66 | C 74,2<br>H 7,4<br>N 6,6 |
| 1.53 | 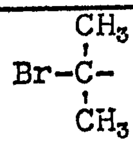 | 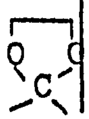 | $CH_3$ | $CH_3$ | H | 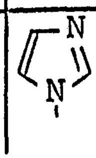 | 164-165 | C 61,86<br>H 6,02<br>N 5,77 | C 61,8<br>H 6,0<br>N 5,6 |

| Verbdgs-Nr. | Z | X | $R_1$ | $R_2$ | $R_3$ | D | Fp.: /°C/ | Analyse: ber.: | gef.: |
|---|---|---|---|---|---|---|---|---|---|
| 1.54 | 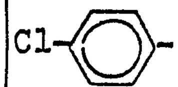 | 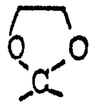 | $CH_3$ | $CH_3$ | H | 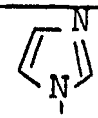 | | | |
| 1.55 | 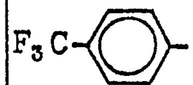 | 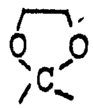 | $CH_3$ | $CH_3$ | H | 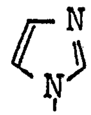 | Oel | C 68,50<br>H 5,35<br>N 5,51 | C 68,1<br>H 5,4<br>N 5,4 |
| 1.56 | 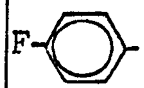 | 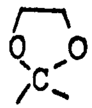 | $CH_3$ | $CH_3$ | H | 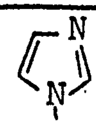 | | | |
| 1.57 | 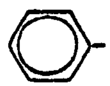 | 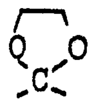 | $CH_3$ | $CH_3$ | H | 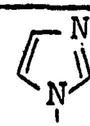 | | | |
| 1.58 | tert.-butyl | 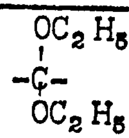 | $CH_3$ | $CH_3$ | H | 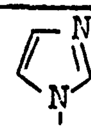 | Oel | C 74,64<br>H 8,50<br>N 6,22 | C 74,50<br>H 8,4<br>N 6,0 |
| 1.59 | tert.-butyl | 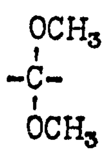 | $CH_3$ | $CH_3$ | H | 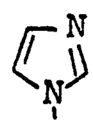 | Oel | C 73,90<br>H 8,11<br>N 6,63 | C 73,7<br>H 8,1<br>N 6,5 |

| Verbdgs-Nr. | Z | X | $R_1$ | $R_2$ | $R_3$ | D | Fp.: [°C] | Analyse: ber.: gef.: |
|---|---|---|---|---|---|---|---|---|
| 1.60 | tert.-butyl | 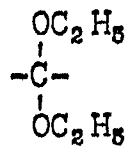 | $-C_2H_5$ | $C_2H_5$ | H | 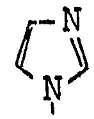 | | |
| 1.61 | tert.-butyl | 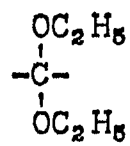 | $-CH(CH_3)_2$ | $-CH(CH_3)_2$ | H | 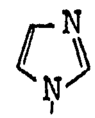 | | |
| 1.62 | 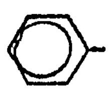 | 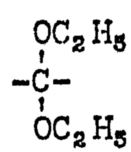 | $CH_3$ | $CH_3$ | H | 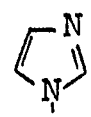 | | |
| 1.63 | 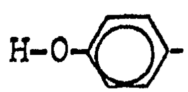 | 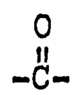 | $CH_3$ | $CH_3$ | H | 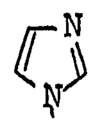 | | |
| 1.64 | tert.-butyl | 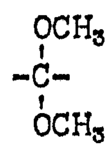 | 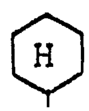 | 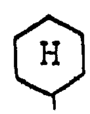 | H | 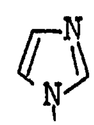 | | |

Bespiel 5

α-Tert.-butyl-4-[2-Chlor-6-methyl-4(imidazol-1-ylmethyl)-phenoxymethyl]-benzylalkohol

Die Suspension aus 4.04 g = 20 mmol Aluminiumisopropylat und 7.92 g = 20 mmol 4-[2-Chlor-6-methyl-4-(imidazol-1-yl-methyl)-phenoxymethyl]-phenyl-tert.-butyl-keton (aus Beispiel 1) in 50 ml Isopropanol wird 10 h auf ca. 80 C erhitzt, wobei über eine 10 cm Vigreux-Kolonne ca. 5 ml Isopropanol/Aceton-Gemisch pro Stunde abdestilliert werden und der Lösungsmittelverlust durch Zugabe von Isopropanol ausgeglichen wird. Danach zieht man i.V. das Lösungsmittel ab, nimmt den Rückstand in 50 ml Wasser auf, versetzt mit 30 ml 2 n $H_2SO_4$ und rührt 30 min. bei Raumtemperatur nach. Mit 2 n $Na_2CO_3$ wird die Lösung auf pH3 gebracht, mit Methylenchlorid ausgeschüttelt und *) aufgearbeitet. Der Rückstand wird in Äther/Diisopropylether aufgenommen und das Kristallisat abgesaugt. Man erhält 5,2 g vom Fp.: 150 - 153°C (65 % d. Th.)

*) wie in Beispiel 2 angegeben

Analyse:

| | | | | |
|---|---|---|---|---|
| $C_{23}H_{27}ClN_2O_2$ | Ber.: | C= 69.25 | Gef.: | C = 68.9 |
| MG: 398,93 | | H= 6,82 | | H = 7.0 |
| | | N= 7,02 | | N = 6.9 |

Beispiel 6

α-Tert.-butyl-4-[2,5-dimethyl-4-(imidazol-1-ylmethyl)-phenoxymethyl]-benzylalkohol

Gemäß Beispiel 5, jedoch unter Verwendung äquivalenter Mengen des Ketons aus Beispiel 2 und Aluminiumisopropylat erhält man 5.3 g (70 % d. Th.)

Analyse:

| | | |
|---|---|---|
| $C_{24}H_{30}N_2O_2$ | Ber.: C = 76,16 | Gef.: C = 76.0 |
| MG: 378.52 | H = 7.99 | H = 7.8 |
| | N = 7.40 | N = 7.3 |

Beispiel 7

α-Tert.-butyl-4-/4-(imidazol-1-ylmethyl)-phenoxymethyl/-benzylalkohol

Zur Lösung von 3.5 g = 10 mmol 4-/4-(Imidazol-1-ylmethyl)-phenoxymethyl/-phenyl-tert.-butylketon in 40 ml Methanol gibt man unter Rühren und Kühlen bei 0°C 0.38 g = 10 mmol Natriumborhydrid portionsweise zu, läßt anschließend auf Raumtemperatur aufwärmen und 15 h stehen. Mit Eisessig wird pH 6 - 7 eingestellt, das Lösungsmittel i.V. abgezogen und wie *) mit Wasser/Methylenchlorid aufgearbeitet. Der Rückstand wird in Ether aufgenommen und das Kristallisat abgesaugt. Man erhält 2,6 g vom Fp.: 130 - 132°C (75 % d. Th.).

*) in Beispiel 2 beschrieben

Analyse:

| | | |
|---|---|---|
| $C_{22}H_{26}N_2O_2$ | Ber.: C = 75.40 | Gef.: C = 75.2 |
| MG: 350.46 | H = 7,48 | H = 7.6 |
| | N = 7,99 | N = 7.9 |

Bei gleicher Arbeitsweise wie vorstehend beschrieben können auch Ethanol, Isopropanol, Propanol, n-Butanol, tert.-Butanol und Methylglykol als Lösungsmittel verwendet werden.

Beispiel 8

Gemäß Beispiel 7, jedoch unter Verwendung äquivalenter Mengen der entsprechenden Verbindungen der Formel I aus Tabelle 1 und Natriumborhydrid können die folgenden, in Tabelle 2 aufgeführten Verbindungen der Formel I hergestellt werden.

Tabelle 2

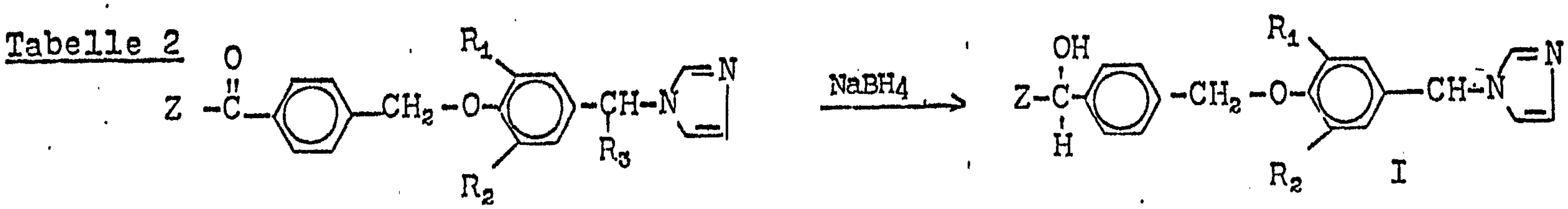


I

| Verbdgs.-Nr. | Z | $R_1$ | $R_2$ | $R_3$ | hergestellt aus | Fp.: [°C] | Analyse: ber.: | gef.: |
|---|---|---|---|---|---|---|---|---|
| 2.1 | tert.-butyl | $CH_3$ | $CH_3$ | H | Beisp. 3a | 126 | C 76,16<br>H 7,99<br>N 7,40 | C 76,1<br>H 8,2<br>N 7,2 |
| 2.2 | tert.-butyl | $CH_3$ | allyl | H | 1.2 | 115-117 | C 77,19<br>H 7,97<br>N 6,92 | C 77,0<br>H 7,9<br>N 6,8 |
| 2.3 | tert.-butyl | $-C_2H_5$ | $-C_2H_5$ | H | 1.5 | 136-138 | C 76,80<br>H 8,43<br>N 6,89 | C 76,9<br>H 8,4<br>N 6,8 |
| 2.4 | tert.-butyl | iso-propyl | iso-propyl | H | 1.3 | 149-150 | C 77,38<br>H 8,81<br>N 6,45 | C 77,1<br>H 8,8<br>N 6,3 |
| 2.5 | tert.-butyl | Cl | allyl | H | 1.7 | 121 | C 70,65<br>H 6,88<br>N 6,59 | C 70,5<br>H 6,8<br>N 6,6 |
| 2.6 | tert.-butyl | $-OCH_3$ | $-OCH_3$ | H | 1.8 | 186 | C 70,22<br>H 7,37<br>N 6,82 | C 69,9<br>H 7,4<br>N 6,7 |

| Verbdgs.-Nr. | Z | $R_1$ | $R_2$ | $R_3$ | hergestellt aus | Fp.: [°C] | Analyse: ber.: | gef.: |
|---|---|---|---|---|---|---|---|---|
| 2.7 | $CH_3$<br>$-C-CH_2-Cl$<br>$CH_3$ | $CH_3$ | $CH_3$ | H | 1.16 | | | |
| 2.8 | $CH_3$<br>$-C-CH_2-F$<br>$CH_3$ | $CH_3$ | $CH_3$ | H | 1.17 | | | |
| 2.9 | $CH_3$<br>$-C-CH_2-CH_3$<br>$CH_3$ | $CH_3$ | $CH_3$ | H | 1.12 | | | |
| 2.10 | $CH_3$<br>$-C-(CH_2)_2-CH_3$<br>$CH_3$ | $CH_3$ | $CH_3$ | H | 1.13 | | | |
| 2.11 | 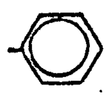 | $CH_3$ | $CH_3$ | H | 1.45 | | | |

| Verbdgs.-Nr. | Z | $R_1$ | $R_2$ | $R_3$ | hergestellt aus | Fp.: [°C] | Analyse: ber.: | gef.: |
|---|---|---|---|---|---|---|---|---|
| 2.12 | 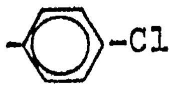 | $CH_3$ | $CH_3$ | H | 1.46 | Oel | C 72,13<br>H 5,82<br>N 6,47 | C 72,1<br>H 5,7<br>N 6,3 |
| 2.13 | 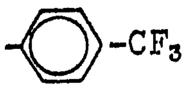 | $CH_3$ | $CH_3$ | H | 1.47 | | | |
| 2.14 | tert.-butyl | $CH_3$ | $CH_3$ | $CH_3$ | 1.9 | | | |
| 2.15 | 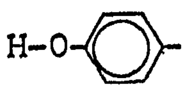 | $CH_3$ | $CH_3$ | H | 1.61 | | | |
| 2.16 | tert.-butyl | 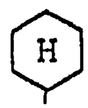 | 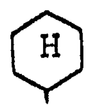 | H | 1.43 | | | |
| 2.17 | tert.-butyl | 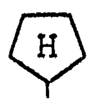 | 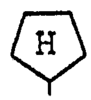 | H | 1.44 | | | |

## HERSTELLUNG EINIGER AUSGANGSSTOFFE DER FORMEL III BZW. III'

### Beispiel 9

a) 4-Methylphenyl-(1-chlor-2,2-dimethyl)-ethylketon

Die Lösung von 0.3 mol 4-Methylphenyl-Magnesiumbromid in 250 ml abs. Tetrahydrofuran (hergestellt aus 8.02 g = 0.33 mol Magnesium und 51.31 g = 0.3 mol 4-Bromtoluol) tropft man unter Rühren bei - 10°C zur Lösung von 36.6 g = 0.24 mol Chlorpivalinsäurechlorid in 150 ml abs. Tetrahydrofuran, läßt auf Raumtemperatur aufwärmen und rührt 1 h nach. Unter Rühren und Kühlen werden bei 20°C 35 ml 2n HCl zugegeben, die organische Phase abgetrennt und i. V. abgezogen. Der Rückstand wird in 100 ml Methylenchlorid aufgenommen, mit 40 ml 2n HCl, dann mit 40 ml 2n $Na_2CO_3$-Lösung gewaschen und wie *) aufgearbeitet. Nach Destillation über eine 10 cm Vigreux-Kolonne erhält man 32 g vom $Kp_{0.4}$ 90 - 93°C (64 % d. Th.).

*) in Beispiel 2 angegeben

b) 4-Brommethylphenyl-(1-chlor-2,2-dimethyl)-ethylketon

Die Suspension aus 16 g = 76 mmol der vorstehenden Verbindung, 13.52 g = 76 mmol N-Bromsuccinimid und 0.5 g Azo-bis-isobutyronitril in 80 ml Tetrachlorkohlenstoff wird unter Rühren 4 h am Rückfluß gekocht. Man saugt das Succinimid ab, wäscht mit $CCl_4$ nach, trocknet die Mutterlauge über $Na_2CO_3$, zieht i. V. das Lösungsmittel ab und destilliert den Rückstand wie vorstehend beschrieben. Man erhält 13.5 g vom $Kp_{0.2}$ 120 - 125°C, GC 87 %ig (53 % d.Th.).

Beispiel 10

a) 2-(4-Methylphenyl)-2-tert.-butyl-1,3-dioxolan (Verbindung der Formel IV)

Die Mischung aus 17.6 g = 0.1 mol 4-Methylphenyl-tert.-butyl-keton, 20 ml n-Butanol, 20 ml Ethylenglykol, 50 ml Benzol und0.5 g p-Toluolsulfonsäure wird 17 h am Wasserabscheider gekocht, die kalte Lösung mit 100 ml Wasser und 20 ml 2n $Na_2CO_3$ gewaschen, *) . Der kristalline Rückstand wird aus Ethanol umkristallisiert. Man erhält 16 g vom Fp.: 66 - 67°C (73 % d. Th.).

*)über $Na_2CO_3$ getrocknet und das Lösungsmittel i.V. abgezogen.

b)2-(4-Brommethylphenyl)-2-tert.-butyl-1.3-dioxolan (Verbindung der Formel III)

Gemäß Beispiel 9 b, jedoch unter Verwendung von 22 g = 0.1 mol der vorstehenden Verbindung und 17.8 g = 0.1 mol N-Bromsuccinimid erhält man 29 g Öl, das nach Zugabe von 30 ml Methanol kristallisiert. Man erhält 19.4 g vom Fp.: 76 - 78°C (65 % d. Th.).

Beispiel 11

Gemäß Beispiel 10 a und b, jedoch unter Verwendung äquivalenter Mengen von entsprechenden Verbindungen der Formel IV und N-Bromsuccinimid werden die folgenden Verbindungender Formel III' hergestellt:

2-(4-Brommethylphenyl)-2-(4-trifluormethylphenyl)-1.3-dioxolan
Fp.: 65 - 67 C
2-(4-Brommethylphenyl)-2-(2-brom-2-methyl-ethyl)-1,3-dioxolan
Fp.: 102 - 104 C
2-(4-Brommethylphenyl)-2-(4-chlorphenyl)-1.3-dioxolan
Fp.: 88 - 89 C
2-(4-Brommethylphenyl)-2-phenyl-1.3dioxolan

Beispiel 12

a) 4-Methylbenzophenondiethylketal

Die Lösung aus 9.8 g = 50 mmol 4-Methylbenzophenon, 8.6 g = 110 mmol Orthoameisensäuretriethylester, 10 ml abs. Ethanol und einem Tropfen konz. Schwefelsäure läßt man 5 Tage bei Raumtemperatur stehen. Anschließend zieht man i. V. das Lösungsmittel ab, löst den Rückstand in 50 ml Methylenchlorid, wäscht mit 30 ml 2n $Na_2CO_3$-Lösung, trocknet über $K_2CO_3$ und zieht i. V. das Methylenchlorid ab. Nach Destillation im Ölpumpenvakuum erhält man 11.1 g vom $Kp_{0.4}$ 125 - 130°C (82 % d. Th.).

b) 4-Brommethylbenzophenondiethylketal

Gemäß Beispiel 9 b werden 10.8 g = 40 mmol der vorstehenden Verbindung mit 7.12 g = 40 mmol N-Bromsuccinimid umgesetzt und aufgearbeitet. Nach Destillation im Ölpumpenvakuum über eine 10 cm Vigreux-Kolonne erhält man 10.5 g vom $Kp_{0.4}$ 155 - 157 C (75 % d. Th.).

Beispiel 13

4-Brommethylphenyl-tert.-butyl-keton-diethylketal

Die Lösung aus 12,75 g = 50 mmol 4-Brommethylphenyl-tert.-butylketon, 8.6 g = 110 mmol Orthoameisensäuretriethylester, 10 ml abs. Ethanol und einem Tropfen konz. Schwefelsäure läßt man 5 Tage bei Raumtemperatur stehen. Anschließend zieht man i.V. das Lösungsmittel ab, löst den Rückstand in 50 ml Methylenchlorid, wäscht mit 30 ml 2n $Na_2CO_3$-Lösung, trocknet über $K_2CO_3$ und zieht i.V. das Methylenchlorid ab. Nach Destillation im Ölpumpenvakuum erhält man 13,2 g vom $Kp_{0.5}$ 119 - 120°C (80 % d.Th.).

Beispiel 14

4-Brommethylphenyl-tert.-butyl-keton-dimethylketal

Gemäß Beispiel 13 werden 4-Brommethylphenyl-tert.-butylketon mit Methanol und Orthoameisensäuretrimethylester umgesetzt und aufgearbeitet. Man erhält 12.3 g vom $Kp_{0.6}$ 115-118°C (82 % d. Th.).

Patentansprüche:

1. Benzylether von Phenol-Mannich-Basen der Formel I

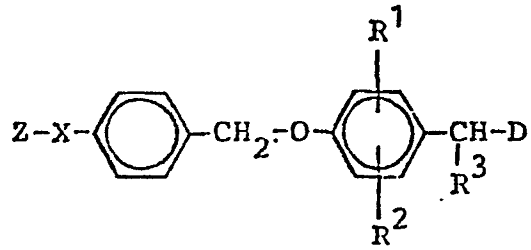

I

in der

$R^1$ und $R^2$ gleich oder verschieden sind und jeweils Wasserstoff, $C_1$-$C_4$-Alkyl, $C_5$-$C_6$-Cycloalkyl, $C_1$-$C_4$-Alkoxy, Allyl oder Halogen bedeuten

$R^3$ Wasserstoff oder $C_1$-$C_4$-Alkyl bedeutet,

D entweder

a) einen Rest der Formel D (a),

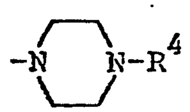

D (a)

in der

$R^4$ Wasserstoff, $C_1$-$C_8$-Alkyl, Allyl, Hydroxyethyl, $C_1$-$C_5$-Alkanoyl, $C_1$-$C_4$-Alkylsulfonyl, $C_1$-$C_4$-Alkoxycarbonyl, Di-($C_1$-$C_4$)-alkylaminocarbonyl oder eine gegebenenfalls 1 oder 2 Substituenten tragende Phenylgruppe bedeutet, wobei die Substituenten gleich oder verschieden sind und jeweils Halogen, $C_1$-$C_4$-Alkyl, $C_1$-$C_4$-Alkoxy oder Trifluormethyl bedeuten, oder

b) einen Pyrrolidin-1-yl-, Piperidin-1-yl-, Morpholin-4-yl-, Pyrazol-1-yl-, Imidazol-1-yl-, 1,2,4-Triazol-1-yl- oder einen Benzimidazol-1-yl-Rest bedeutet,,

X 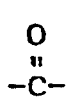 , 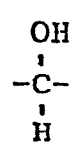 , 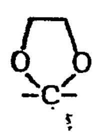 oder 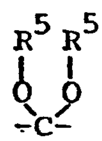 bedeutet,

wobei $R^5$ $C_1$-$C_4$-Alkyl ist,

Z a) einen Rest der Formel Za

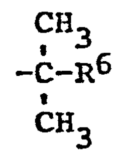 Z(a)

in der $R^6$ H, $C_1$-$C_4$-Alkyl, Halogen oder $-CH_2-$Halogen bedeutet, oder

b) eine gegebenenfalls 1 oder 2 Substituenten tragende Phenylgruppe bedeutet, wobei die Substituenten gleich oder verschieden sind und jeweils Halogen, Hydroxy, $C_1$-$C_4$-Alkyl, $C_1$-$C_4$-Alkoxy oder Trifluormethyl bedeuten,

und ihre physiologisch verträgliche Salze mit Säuren.

2. Benzylether von Phenol-Mannich-Basen und deren physiologisch verträgliche Salze gemäß Anspruch 1, dadurch gekennzeichnet, daß D Imidazol-1-yl ist, $R^1$ und $R^2$ gleich oder verschieden sind und jeweils Methyl, Ethyl oder Chlor bedeuten, $R^3$ Wasserstoff, X die $\underset{\overset{\|}{O}}{-C-}$ oder $\overset{OH}{\underset{H}{-C-}}$ Gruppen und Z tert.-Butyl bedeutet.

3. Verfahren zur Herstellung von Verbindungen gemäß Anspruch 1, dadurch gekennzeichnet, daß man eine Verbindung der Formel II

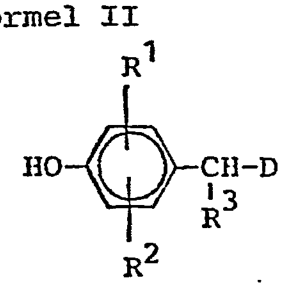 II

in der $R^1$, $R^2$, $R^3$ und D die zu Formel I angegebenen Bedeutungen haben, mit einer Verbindung der Formel III

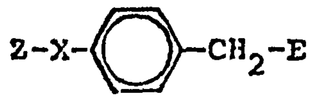

III

in der X und Z die zu Formel I angegebenen Bedeutungen haben und E Halogen bedeutet, umsetzt, gegebenenfalls erhaltene Verbindungen, worin X die $-\overset{\|}{\underset{O}{C}}-$ Gruppe bedeutet, zu Verbindungen der Formel I, worin X = $-\underset{H}{\overset{OH}{\overset{|}{\underset{|}{C}}}}-$ bedeutet, reduziert oder zu Verbindungen, worin X = 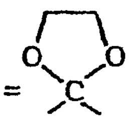 oder 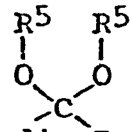 in an sich bekannter Weise ketalysiert, und die Reaktionsprodukte gegebenenfalls mit Säuren in physiologisch verträgliche Säureadditionssalze überführt.

4. Pharmazeutisches Präparat enthaltend eine Verbindung gemäß Anspruch 1.

5. Verfahren zur Herstellung eines pharmazeutischen Präparates, dadurch gekennzeichnet, daß man eine Verbindung gemäß Anspruch 1 in eine geeignete Darreichungsform bringt.

6. Verwendung von Verbindungen gemäß Anspruch 1 zur Beeinflussung des Serumlipoproteinspektrums.

7. Verbindungen der Formel III'

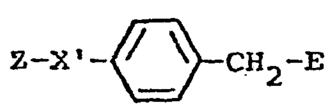

III'

worin Z die zu Formel I und E die zu Formel III angegebene Bedeutungen haben und X' 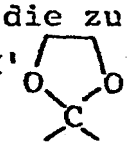 oder 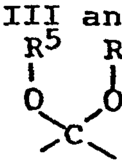 wobei $R^5$ $C_1$-$C_4$-Alkyl ist, bedeutet.

8. Verfahren zur Herstellung von Verbindungen der Formel III' gemäß Anspruch 7, dadurch gekennzeichnet, daß man

a) eine Verbindung der Formel IV

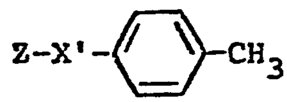

IV

worin Z die zu Formel I und X' die zu Formel III' angegebenen Bedeutungen haben, in an sich bekannter Weise halogeniert oder

b) eine Verbindung der Formel V

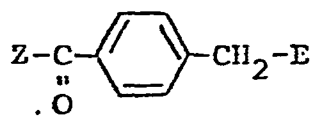

V

worin Z die zu Formel I u.E die zu Formel III angegebenen Bedeutungen haben, mit Ethylenglykol oder einem Alkohol $R^5$-OH, wobei $R^5$ $C_1$-$C_4$-Alkyl bedeutet, umsetzt.

Patentansprüche für Österreich

1. Verfahren zur Herstellung von Benzylethern von Phenol-Mannich-Basen der Formel I

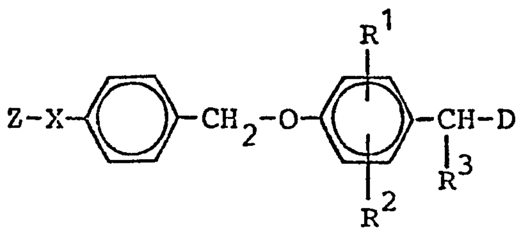

I

in der

$R^1$ und $R^2$ gleich oder verschieden sind und jeweils Wasserstoff, $C_1$-$C_4$-Alkyl, $C_5$-$C_6$-Cycloalkyl, $C_1$-$C_4$-Alkoxy, Allyl oder Halogen bedeuten

$R^3$ Wasserstoff oder $C_1$-$C_4$-Alkyl bedeutet,

D entweder

a) einen Rest der Formel D (a),

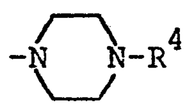

D (a)

in der

$R^4$ Wasserstoff, $C_1$-$C_8$-Alkyl, Allyl, Hydroxyethyl, $C_1$-$C_5$-Alkanoyl, $C_1$-$C_4$-Alkylsulfonyl, $C_1$-$C_4$-Alkoxycarbonyl, Di-($C_1$-$C_4$)-alkylaminocarbonyl oder eine gegebenenfalls 1 oder 2 Substituenten tragende Phenylgruppe bedeutet, wobei die Substituenten gleich oder verschieden sind und jeweils Halogen, $C_1$-$C_4$-Alkyl, $C_1$-$C_4$-Alkoxy oder Trifluormethyl bedeuten, oder

b) einen Pyrrolidin-1-yl-, Piperidin-1-yl-, Morpholin-4-yl-, Pyrazol-1-yl-, Imidazol-1-yl-, 1,2,4-Triazol-1-yl- oder einen Benzimidazol-1-yl-Rest bedeutet,

X 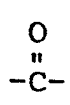 , 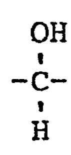 , 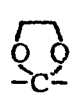 oder 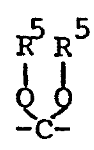 bedeutet,

wobei $R^5$ $C_1$-$C_4$-Alkyl ist,

Z a) einen Rest der Formel Z(a)

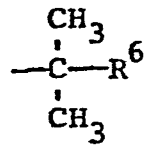 Z (a)

in der $R^6$ H, $C_1$-$C_4$-Alkyl, Halogen oder $-CH_2$-Halogen bedeutet, oder

b) eine gegebenenfalls 1 oder 2 Substituenten tragende Phenylgruppe bedeutet, wobei die Substituenten gleich oder verschieden sind und jeweils Halogen, Hydroxy, $C_1$-$C_4$-Alkyl, $C_1$-$C_4$-Alkoxy oder Trifluormethyl bedeuten,

sowie ihren physiologisch verträglichen Salzen mit Säuren, dadurch gekennzeichnet, daß man eine Verbindung der Formel II

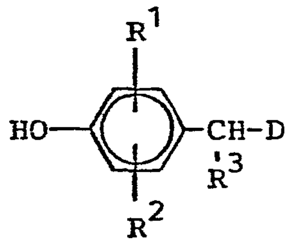 II

in der $R^1$, $R^2$, $R^3$ und D die zu Formel I angegebenen Bedeutungen haben, mit einer Verbindung der Formel III

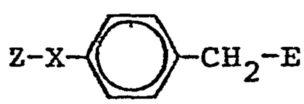 III

in der X und Z die zu Formel I angegebenen Bedeutungen haben und E Halogen bedeutet, umsetzt, gegebenenfalls erhaltene Verbindungen, worin X die 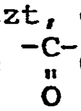 Gruppe bedeutet,

zu Verbindungen der Formel I, worin X = 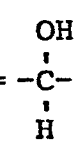 bedeutet,

reduziert oder zu Verbindungen, worin

X = 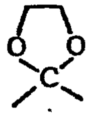 oder 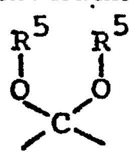 in an sich bekannter Weise

ketalysiert, und die Reaktionsprodukte gegebenenfalls

0104572
HOE 82/F 190

mit Säuren in physiologisch verträgliche Säureadditionssalze überführt.

2. Verfahren gemäß Anspruch 1, dadurch gekennzeichnet, daß man durch Verwendung entsprechend substituierter Ausgangsverbindungen Verbindungen der Formel I, worin D Imidazol-1-yl ist, $R^1$ und $R^2$ gleich oder verschieden sind und jeweils Methyl, Ethyl oder Chlor bedeuten, $R^3$ Wasserstoff, X die $\underset{\underset{O}{\|}}{-C-}$ oder $\overset{\overset{OH}{|}}{\underset{\underset{H}{|}}{-C-}}$ Gruppen und Z tert.-Butyl bedeutet, oder deren physiologisch verträgliche Salze herstellt.

3. Verfahren zur Herstellung von Verbindungen der Formel III'

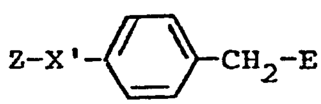

III'

worin
Z die zu Formel I und E die zu Formel III angegebenen Bedeutungen haben und X' 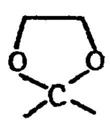 oder 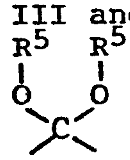 wobei $R^5$ $C_1$–$C_4$-Alkyl ist,
bedeutet, dadurch gekennzeichnet, daß man

a) eine Verbindung der Formel IV

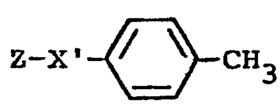

IV

worin Z die zu Formel I und X' die zu Formel III' angegebenen Bedeutungen haben, in an sich bekannter Weise halogeniert oder

b) eine Verbindung der Formel V

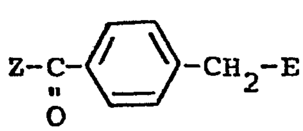

V

worin Z die zu Formel I u.E. die zu Formel III angegebenen Bedeutungen haben, mit Ethylenglykol oder einem Alkohol $R^5$-OH, wobei $R^5$ $C_1$-$C_4$-Alkyl bedeutet, umsetzt.

**0104572**

Nummer der Anmeldung

Europäisches Patentamt

## EUROPÄISCHER TEILRECHERCHENBERICHT,

der nach Regel 45 des Europäischen Patentübereinkommens für das weitere Verfahren als europäischer Recherchenbericht gilt

EP 83109251.5

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A,D | DE - A1 - 3 001 762 (PFIZER CORP.)<br>* Ansprüche 1,8,10 *<br>-- | 1,3,4,7 | C 07 D 233/60<br>C 07 D 235/06<br>C 07 D 231/12<br>C 07 D 249/08<br>C 07 D 295/08<br>C 07 D 317/22<br>C 07 D 405/12<br>C 07 C 43/313<br>A 61 K 31/40<br>A 61 K 31/41<br>A 61 K 31/415<br>A 61 K 31/445<br>A 61 K 31/495<br>A 61 K 31/535 |
| A,D | DE - A1 - 2 510 781 (SCHERING AG)<br>* Ansprüche 1,49,50 *<br>-- | 1,3,4,7 | |
| A | GB - A - 1 489 711 (SCIENCE UNION ET CIE)<br>* Ansprüche 1,11; Seite 2, Zeilen 6-15 *<br>-- | 1,4 | |
| A | FR - A - 1 601 591 (R. ARIES)<br>* Ansprüche 1,5; Seite 1, Zeilen 35-40 *<br>-- | 1,3 | **RECHERCHIERTE SACHGEBIETE (Int. Cl. 3)** |
| A | US - A - 4 008 327 (F.G. KATHAWALA)<br>* Formel I *<br>---- | 1,7 | C 07 C 43/00<br>C 07 D 233/00<br>C 07 D 235/00<br>C 07 D 231/00<br>C 07 D 249/00<br>C 07 D 295/00<br>C 07 D 317/00<br>C 07 D 405/00 |

### UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-5,7,8

Unvollständig recherchierte Patentansprüche: –

Nicht recherchierte Patentansprüche: 6

Grund für die Beschränkung der Recherche:

Verfahren zur therapeutischen Behandlung des menschlichen oder tierischen Körpers, Art. 52(4) EPÜ

| Recherchenort | Abschlußdatum der Recherche | Prüfer |
|---|---|---|
| WIEN | 18-12-1983 | BRUS |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1505.1 03 82